# EUROPEAN PATENT SPECIFICATION

(11) **EP 2 804 588 B1**
(45) Date of publication and mention of the grant of the patent: **06.09.2017**
(21) Application number: 12700675.7
(22) Date of filing: 17.01.2012
(51) Int. Cl.: A61K 9/14, A61K 9/20, A61K 31/137

(54) **METHOD FOR PRODUCING CINACALCET COMPOSITIONS FOR DIRECT TABLETING**
VERFAHREN ZUR HERSTELLUNG VON CINACALCETZUSAMMENSETZUNGEN ZUR DIREKTTABLETTIERUNG
PROCÉDÉ DE PRÉPARATION DE COMPOSITIONS DE CINACALCET DESTINÉES À LA FABRICATION DIRECTE DE COMPRIMÉS

(43) Date of publication of application: 26.11.2014
(73) Proprietor: Zentiva Saglik Urunleri San. Ve Tic. A.S., 39780 Kirklareli (TR)
(72) Inventor: GURLEYENDAG, Bahar, 39780 Kirklareli (TR); ORAN, Umut, 39780 Kirklareli (TR); BITIK, Naci, 39780 Kirklareli (TR); ADIYAMAN, Mustafa, 39780 Kirklareli (TR)
(74) Representative: Sevinç, Erkan
(86) International application number: PCT/EP2012/050661
(87) International publication number: WO 2013/107503

(56) References cited:
- WO-A1-2005/034928
- WO-A1-2010/086129
- WO-A2-2008/027522
- WO-A2-2010/034497

## Description

### Field of the Invention

The present invention relates to a novel method for producing pharmaceutical compositions of cinacalcet or pharmaceutically acceptable salts thereof comprising one or more pharmaceutically acceptable excipients. More particularly, the invention relates to a direct compression method for producing cinacalcet compositions having improved compressibility properties and better dissolution profile as compared to the dry processes of the art. The invention further relates to a method for manufacturing of such compositions in tablet forms.

### Background of the Invention

Cinacalcet hydrochloride is a calcium receptor-active compound that is currently marketed under the trade name SENSIPAR (in U.S.) and MIMPARA (in EUROPE) for the treatment of hyperparathyroidism in patients with chronic kidney disease on hemodialysis which is described in U.S. Patent No. 6,001,884.

Calcium receptor-active compounds, especially Cinacalcet *inter alia* is known with its hardly soluble nature in water and soluble behavior in some organic solvents as reported in WO 2005/034928-A1. It was also reported that cinacalcet has solubility in water of less than about 1 µg/ml at neutral pH, while this value can reach up to 1.6 mg/ml when the pH ranges from about 3 to 5. WO 2005/034928 further describes the wet granulation method for rapid dissolution formulation of Cinacalcet HCl, wherein the composition has a dissolution profile in 0.05N HCl from about 50% to about 125% of a target amount of the calcium receptor-active compound being released from the composition no later than 30 minutes. The aspects of the present invention eliminate the disadvantages of wet granulation mentioned above, and furthermore it provides a method of controlling the dissolution rate of the formulation comprising an effective dosage amount of calcium receptor-active compound.

It is therefore, currently a challenge of those skilled in the art to increase solubility of Cinacalcet without deteriorating its physical properties.

To achieve this object, formulations of cinacalcet are generally prepared by wet or melt granulation techniques as disclosed, for instance in WO 2005/034928-A1 and WO 2011/047837-A2. These documents, although directed to solve solubility problems of solid oral forms of cinacalcet, have the common proposal to sub-micronize the overall composition to a particle size range below 500 µm. Likewise, US 2011/0287065 discloses a cinacalcet composition having an overall particle size distribution of less than 2000 nm in order to achieve better solubility and bioavailability. Disclosure, however is silent with regard to the preparation method of the solid oral compositions, especially of the tablets containing cinacalcet.

US 2010/0168247-A1 discloses immediate and controlled release pharmaceutical formulations of cinacalcet, as well as methods for manufacturing such formulations. It was proposed to apply wet granulation for the preparation of a solid composition involving cinacalcet and a coating agent, such as Eudragit®, wherein the particle size was arranged to be below 400 µm. Similarly, WO 2010/034497-A2 discloses solid compositions prepared from flakes wherein the size (D90) of cinacalcet particles ranges from 200 to 800 µm, and high level of disintegrant content as high as 10% to 30% (w/w) is required for providing an acceptable composition.

The stated art as provided hereinabove, basically aims to provide solid compositions with better solubility. The inventors of the present invention, however noted that properties of compressibility of cinacalcet need also to be improved, as the existing methods for production of tablets, while improving solubility, reveal considerable problems in terms of sticking, clusters, pelletization and non-uniform die filling. Hence, the compositions of common general art are generally not suitable for direct compression in manufacturing of tablets. It is therefore highly preferable in conventional methods to use wet granulation before subjecting the particles to tablet compression.

The inventors also noted that using wet granulation has disadvantageous effects on heat and moisture sensitive drugs. The large number of processing steps and processing time are also problems causing high level manufacturing costs. Wet granulation has also been known to reduce the compressibility of some pharmaceutical excipients such as microcrystalline cellulose.

To this end, elimination of the classical wet granulation steps in preparation of compressible powders of cinacalcet and providing the tablets *per se* will be a considerable development in the formulation science as may be appreciated by those skilled people in the art.

It is therefore, one of the objects of the present invention to eliminate complicated and costly steps of wet granulation in production of compressible cinacalcet compositions.

A further object of the present invention is to eliminate adverse effects of wet treatment of cinacalcet compositions before compression.

Another object of the present invention is to provide a method for producing cinacalcet compositions that results in modified particles suitable to be directly compressed.

Still a further object of the present invention is to provide a novel method of producing the aforesaid composition and resulting tablets thereof without suffering of the drawbacks of the prior art.

These objects have been achieved through a method according to claim 1. Further objects and related advantages of the present invention will be apparent with the below description along with the appended claims.

### Summary of the Invention

Embodiments of the present invention provide a novel direct compression method for the production of pharmaceutical compositions of cinacalcet as well as a further step of producing oral tablet forms thereof, which are very suitable for direct compression and is eliminating the complicated and costly procedures of the prior art. The method comprises initially mixing of cinacalcet or a pharmaceutically acceptable salts thereof having particle size distribution - d(90) of less than 150 µm with a diluent, and thereby providing a core composition having an overall particle size profile of less than 355 µm; the method further comprises separately selecting fillers having bulk density of ranging from 350 to 650 g/l, and mixing the same with the aforesaid core composition such that bulk density of the overall composition is greater than 400 g/l. The novel method may further comprise addition of other excipients and directly compressing the resulting powder into oral tablets.

### Detailed Description of the Invention

This invention provides a new direct compression method of producing compositions of cinacalcet or pharmaceutically acceptable salts thereof having improved compressibility and solubility properties for successfully applying direct compression and tableting. The invention further relates to a method for the preparation of the tablet forms of cinacalcet according to the present invention.

Direct compression is regarded as a relatively quick process where the powdered materials are compressed directly without changing the physical and chemical properties of the drug. The active ingredient(s), direct compression excipients and other auxiliary substances, such as glidants and lubricants are blended before being compressed into tablets.

It is known generally that the advantages of direct compression include few manufacturing steps involved, physical stability and elimination of heat and moisture. According to a first aspect of the present invention, there is provided a novel method for providing a composition of cinacalcet in dry form, for use in direct compression of tablets, comprising the following steps:
- preparing a core composition involving mixing particles of Cinacalcet (or its pharmaceutically acceptable salts) having particle size distribution -d(90) value of less than 150 µm together with at least one diluent,
- sieving the core composition to obtain an overall particle size profile of less than 355 µm,
- separately selecting other fillers to have a bulk density of ranging from 350 to 650 g/l, and mixing the same with the core composition in substantially dry conditions such that bulk density of the overall composition is greater than 400 g/l.

In this aspect, the inventors noted that major drawbacks of known compositions prepared by the conventional processes can be eliminated with the above method while solubility still remains as good as those conventional wet processes.

In the context of the present invention the term "cinacalcet" implies base form of the active material or any pharmaceutically acceptable salt thereof. Cinacalcet hydrochloride is, however mostly preferred.

The specific particle size of cinacalcet in the method described above found to be advantageous for improving solubility of the resulting powder. It was also found particularly advantageous for overcoming the flowability problems and sticking at punches because cinacalcet, normally has a great tendency to stick to punches during tableting and to form clusters in contact with dissolution media limiting its dissolution. On the other hand, the inventors found also that particle size distribution-d(90) of cinacalcet which is below 30 µm may not be desirable because of recurring sticking problem which may only be solved by adding of extra lubricants, for instance up to the 2% (w/w) of the overall composition compromising dissolution. Thus, in a particular embodiment, the cinacalcet particles in the core composition have the particle size distribution profile of 30 µm < d(90) < 150 µm.

Within the above composition designed for direct tableting, cinacalcet is initially brought together with a diluent for desired compressibility features. This step is particularly for homogenizing and isolating the cinacalcet particles from rest of the composition which was noted to be very useful to eliminate clustering and sticking problems of conventionally known dry processes. Therefore, the aforesaid step is regarded as an essential element of the present invention *inter alia* defined herein. A further essential element is to provide said core composition to have a maximum particle size of 355 µm in order to avoid the forming of cinacalcet clusters by the dilution of active substance. This can be achieved by mixing of a diluent powder having particles of less than 355 µm with the aforesaid cinacalcet powders. Alternatively, one skilled in the art would appreciate that overall core composition may also be sieved through a suitable mesh to obtain particles of max. 355µm. The particle size of the active substance and diluent, i.e. core composition is preferably arranged to have particles with sizes maximum of 250 µm. As may be appreciated by those skilled in the art any material known with the sole function of dilution may be used as a diluent. It is, however preferable for the diluent to have specific advantages such as direct compressibility and hygroscopicity. Therefore, bearing these properties, pregelatinized starch is mostly preferred.

According to the two-step method as disclosed in claim 1, the selected fillers must have a bulk density ranging from 350 to 650 g/l for the sake of better compressibility. The second step may, nevertheless comprise addition of further excipients besides the specified filler materials.

Therefore, the composition, which is very suitable for direct compression according to the first aspect of the present invention may have additional excipients selected from the group consisting of binders, disintegrants, lubricants and glidants as conventionally known in the pharmaceutical industry, with the proviso of bulk density of the final mixture would be above 400 g/l.

The binder material may for instance be copovidone, although many other alternatives are available in the art. Binders are commonly used class of excipients in tablets. Binders are the agents, which impart cohesive qualities to the powdered material. The ratio of binder in direct compression may affect the disintegration time of tablets in case of using excess amount or may affect the compressibility of tablets in case of using an insufficient amount. As an aspect of the present invention, binder content is used greater than 4%.

In case of using the binder less than 4% in the formulation, capping can occur during the compression. Preferably, the binder is included in the composition in an amount ranging from 5% to 10% (w/w).

In the context of the present invention preferred glidant may for instance be colloidal anhydrous silica. On the other hand, the disintegrant may for instance be croscarmellose sodium or crospovidone.

Magnesium stearate is the excipient that may be used in the formulation as lubricant. Adding of magnesium stearate is generally the final step of the preparing of cinacalcet granules.

According to the first aspect above, the invention preferably involves co-processed excipients as filler materials which excipients are found to be very advantageous for improving flowability and other compressibility properties of the present tableting composition. StarLac® is one of the preferable co-processed excipients consisting of 85 % α-lactose monohydrate [Ph.Eur./USP-NF] and 15 % maize starch [Ph.Eur./USP-NF] dry matter. Prosolv® is also a high functionality co-processed excipient consisting of 98% microcrystalline cellulose [Ph.Eur./USP-NF] and 2% colloidal silica [Ph.Eur./USP-NF]. Lactose-based directly compressible excipients can be used as fillers supported by microcrystalline cellulose. A further preferred excipient is FlowLac® which is an excipient of spray-dried α-lactose monohydrate [Ph.Eur./USP-NF] designed for direct compression. Among these, StarLac® and Prosolv® are particularly preferred.

Preferred formulations based on the composition of the present invention can be prepared as disclosed in the examples. The excipients may, however be modified such that the essential limitations (i.e. bulk density, particle sizes) of the first aspect explained above would be satisfied. It is an essential element in the context of the invention that overall bulk density of the composition is arranged to be above 400 g/l.

A preferred formulation that can be prepared in the context of the present invention is set out as follows:

| **Ingredients** | **Preferred amount based on total composition % (w/w)** | **Usable amount based on total composition % (w/w)** |
|---|---|---|
| Cinacalcet HCl | 18.6 | 10 - 40% |
| Pregeletized Starch | 20.0 | 10 - 40% |
| Colloidal anhydrous silica | 1.0 | 0,2 - 2% |
| Crospovidone | 4.0 | 2 - 8% |
| Copovidone | 7.0 | 5 - 10% |
| Prosolv SMCC | 10.0 | 5 - 25% |
| Starlac | 40.0 | 20 - 60% |
| Magnesium Stearate | 0,5 | 0,1 - 2,0% |

The inventors report that amounts of the fillers are of particular importance, and formulation may exhibit disadvantageous properties in terms of compressibility when amounts of the fillers deviate from the specified ranges. In a further aspect of the present invention even 5 to 10% amounts of binder may be sufficient to obtain desired properties of compressibility. The disintegrant ratios of the formulation are linearly related to the obtained dissolution profiles. The inventors further noted that crospovidone may be used as disintegrant, for instance in an amount as low as 2%, and preferably about 4% (w/w) whereas the prior art compositions use disintegrants, and particularly crospovidone in an amount generally more than 20% (w/w) due to the preferred technologies that were generally conventional granulation techniques.

According to a second aspect of the present invention, direct compression is applied directly to the dry composition and specific formulations according to the present invention in order to produce pharmaceutical tablets. Flowability and bulk density are critical parameters for directly compressible powders, wherein flowability is required for uniform die filling, and bulk density is required to ensure good compressibility. The present invention provides improved bulk density of a tableting powder, capable of being directly compressed into a tablet having convenient hardness, rapid disintegration ability, and acceptable dissolution rate.

The method as presented hereinabove is advantageous for obtaining desired dissolution profile without forming clusters. The obtained process avoids the forming of water-borne API clusters at the dissolution medium. It is assumed that sieving of the cinacalcet compound with at least one pharmaceutically acceptable diluent and limiting the particle sizes of active material and excipients to efficient ranges enhanced the overall dissolution rate of the composition.

The invention hereby provides a pharmaceutical composition comprising an effective dosage amount of cinacalcet compound, wherein the composition has a dissolution profile in 900 ml 0.05 N HCl, measured according to a dissolution test conducted in United States Pharmacopeia (USP)- National Formulary (NF) (USP 34/NF 29), chapter 711 using a USP 2 apparatus at a temperature of 37°C ±0.5 °C and a rotation speed of 75 r.p.m, which comprises from about %75 to about 110% of a target amount of calcium receptor-active compound being released from the composition no later than about 30 minutes from the start of the test. The comparative tests of the film coated tablets according to the present invention and prior art are provided in Example IV hereinbelow.

### Example I

### Preparation of oral tablets comprising Cinacalcet HCl

### Preparation of Tableting Powder

Cinacalcet HCl particles having d(90) of less than 150 µm were provided. Cinacalcet HCl was sieved together with Pregelatinized Starch through a 250 µm sieve. Other ingredients were sieved through a 500 µm sieve individually. Cinacalcet HCl and pregelatinized starch were blended together with colloidal anhydrous silica, copovidone, crospovidone, Prosolv® and Flowlac®. Magnesium stearate was then added to the powder mixture and final mixture was continued to be blended.

### Tableting

The final mixture was then compressed into tablets in a rotary tableting machine. While the tablet hardness was around 160 N, the disintegration time was around 6 minutes. The tablets obtained with the procedure above were having the following composition.

**Table I. Formulation according to Example I**

| **INGREDIENTS** | **UNIT FORMULA (mg / tabl.)** |
|---|---|
| Cinacalcet HCl | 99,14 |
| Pregelatinized Starch | 108,00 |
| Colloidal anhydrous silica | 5,40 |
| Copovidone | 37,80 |
| Crospovidone | 21,60 |
| Prosolv | 54,00 |
| Flowlac | 211,36 |
| Magnesium Stearate | 2,70 |

### Example II

### Preparation of oral tablets comprising Cinacalcet HCl

### Preparation of Tableting Powder

Cinacalcet HCl particles having d(90) of less than 150 µm were provided. Cinacalcet HCl was sieved together with Pregelatinized Starch through a 250 µm sieve. Other ingredients were sieved through a 500 µm sieve individually. Cinacalcet HCl and pregelatinized starch were blended together with colloidal anhydrous silica, copovidone, crospovidone, and starlac®. Magnesium stearate was then added to the powder mixture and final mixture was continued to be blended.

### Tableting

The final mixture was then compressed into tablets in a rotary tableting machine. While the tablet hardness was around 170 N, the disintegration time was about 9 minutes. The tablets obtained with the procedure above were having the following composition:

**Table II. Formulation according to Example II**

| **INGREDIENTS** | **UNIT FORMULA (mg / tabl.)** |
|---|---|
| Cinacalcet HCl | 99,14 |
| Sta rlac | 327,46 |
| Pregelatinized Starch | 54,00 |
| Colloidal anhydrous silica | 5,40 |
| Copovidone | 37,80 |
| Croscarmellose Sodium | 10,80 |
| Magnesium Stearate | 5,40 |

### Example III

### Preparation of oral tablets comprising Cinacalcet HCl

### Preparation of Tableting Powder

Cinacalcet HCl particles having d(90) of less than 150 µm were provided. Cinacalcet HCl was sieved together with Pregelatinized Starch through a 355 µm sieve. Other ingredients were sieved through a 500 µm sieve individually. Cinacalcet HCl and pregelatinized starch were blended together with colloidal anhydrous silica, copovidone, crospovidone, prosolv® and starlac®. Magnesium stearate was then added to the powder mixture and final mixture was continued to be blended.

### Tableting

The final mixture was then compressed into tablets in a rotary tableting machine. While the tablet hardness was around 150 N, the disintegration time was about 2 minutes, which is comparable to the originator's product. The tablets obtained with the procedure above were having the following composition.

**Table III. Formulation according to Example III**

| **INGREDIENTS** | **UNIT FORMULA (mg / tabl.)** |
|---|---|
| Cinacalcet HCl | 99,14 |
| Pregelatinized Starch | 108,00 |
| Sta rlac | 222,16 |
| Colloidal anhydrous silica | 5,40 |
| Copovidone | 37,80 |
| Croscarmellose Sodium | 10,80 |
| Prosolv | 54,00 |
| Magnesium Stearate | 2,70 |

### Example IV

### Comparative tests of dissolution profile

90 mg film coated tablet as sold under the tradename of Mimpara® was subjected to dissolution tests parallel to an oral tablet as prepared according to Example III. Comparative Table IV shows the results of the parallel tests for intelligibility of the dissolution behaviors of the both products.

**Table IV. Comparative results of the dissolution tests FDA Medium (0.05 N HCl, 900 ml, 75 rpm)**

| **Time (minutes)** | **Mimpara® 90 mg Film-coated Tablet** | **Cinacalcet 90 mg Film-coatedTablet (Example III)** |
|---|---|---|
| **5** | 56 | 61 |
| **10** | 75 | 80 |
| **15** | 86 | 90 |
| **20** | 91 | 94 |
| **30** | 95 | 99 |
| **45** | 99 | 100 |
| **60** | 100 | 100 |

It was clearly observed that dissolution profile of the composition according to the present invention is fully comparable and even slightly better than the reference product, although it was prepared by a direct compression technique without any wetting agents. The tablets as prepared in Examples I-III were also excellent in compressibility by virtue of the novel composition of the present invention.

## Claims

1. A direct compression method for producing a pharmaceutical composition of cinacalcet or pharmaceutically acceptable salts thereof, comprising the steps of:
preparing a core composition involving mixing of Cinacalcet particles having a particle size distribution -d(90) value of less than 150 µm together with at least one diluent,
sieving the core composition to obtain overall particle size profile of less than 355 µm,
separately selecting filler materials to have a bulk density of ranging from 350 to 650 g/l, said filler materials are present in an amount ranging from 20-60% by weight of the overall composition, and mixing the same with the core composition in substantially dry conditions such that bulk density of the overall composition is greater than 400 g/l.

2. A direct compression method according to claim 1 wherein the method further comprises direct compression of the resulting powders into an oral tablet form.

3. A direct compression method according to claim 1 or 2 wherein the cinacalcet particles have particle size distribution - d(90) of more than 30 µm.

4. A direct compression method according to claim 1 or 2 wherein the cinacalcet particles have particle size distribution - d(90) of less than 30 µm, and the composition further comprises a lubricant up to the 2% (w/w) of the overall composition.

5. A direct compression method according to claim 1 or 2 wherein overall particle sizes within the core composition are less than 250 µm.

6. A direct compression method according to claim 1 or 2 wherein the diluent is pregelatinized starch.

7. A direct compression method according to claim 1 or 2 wherein the other excipients are selected from the group consisting of fillers, binders, disintegrants, lubricants, glidants and co-processed excipients.

8. A direct compression method according to claim 7 wherein the binder is available in an amount ranging from 5% to 10%.

9. A direct compression method according to claim 7 wherein the filler is selected from the group consisting of StarLac®, FlowLac® and Prosolv®.

10. A direct compression method according to any of the preceding claims wherein the composition comprises the final formulation as set out below:
| **Ingredient** | **Amount based on total composition % (w/w)** |
|---|---|
| Cinacalcet HCl | 10 - 40% |
| Pregeletized Starch | 10 - 40% |
| Colloidal anhydrous silica | 0,2 - 2% |
| Crospovidone | 2 - 8% |
| Copovidone | 5 - 10% |
| Prosolv SMCC | 5 - 25% |
| Starlac | 20 - 60% |
| Magnesium Stearate | 0,1 - 2,0% |

## Patentansprüche

1. Direktverpressungsverfahren zur Herstellung einer pharmazeutischen Zusammensetzung von Cinacalcet oder pharmazeutisch annehmbaren Salzen davon, wobei das Verfahren die folgenden Schritte umfasst:
Herstellen einer Kernzusammensetzung durch Vermischen von Cinacalcetteilchen mit einer Korngrößenverteilung eines -d(90)-Werts von unter 150 µm mit mindestens einem Verdünnungsmittel,
Sieben der Kernzusammensetzung zum Erhalt eines Gesamtkorngrößenprofils von unter 355 µm,
separates Auswählen von Füllmaterialien zum Erzielen einer Schüttdichte im Bereich von 350 bis 650 g/l, wobei die Füllmaterialien in einer Menge im Bereich von 20 bis 60 Gew.-% der Gesamtzusammensetzung vorhanden sind, und Mischen dieser mit der Kernzusammensetzung unter im Wesentlichen trockenen Bedingungen, so dass die Schüttdichte der Gesamtzusammensetzung über 400 g/l liegt.

2. Direktverpressungsverfahren nach Anspruch 1, bei dem das Verfahren des Weiteren die Direktverpressung der erhaltenen Pulver zu einer Oraltablette umfasst.

3. Direktverpressungsverfahren nach Anspruch 1 oder 2, bei dem die Korngrößenverteilung eines -d(90)-Werts der Cinacalcetteilchen über 30 µm liegt.

4. Direktverpressungsverfahren nach Anspruch 1 oder 2, bei dem die Korngrößenverteilung eines -d(90)-Werts der Cinacalcetteilchen unter 30 µm liegt, und die Zusammensetzung des Weiteren ein Schmiermittel zu bis zu 2 Gewichtsprozent der Gesamtzusammensetzung umfasst.

5. Direktverpressungsverfahren nach Anspruch 1 oder 2, bei dem die Gesamtteilchengrößen in der Kernzusammensetzung unter 250 µm liegen.

6. Direktverpressungsverfahren nach Anspruch 1 oder 2, bei dem das Verdünnungsmittel Quellstärke ist.

7. Direktverpressungsverfahren nach Anspruch 1 oder 2, bei dem die anderen Hilfsstoffe ausgewählt sind aus der Gruppe bestehend aus Füllmitteln, Bindemitteln, Sprengmitteln, Schmiermitteln, Gleitmitteln und co-prozessierten Hilfsstoffen.

8. Direktverpressungsverfahren nach Anspruch 7, bei dem das Bindemittel in einer Menge im Bereich von 5 % bis 10 % vorhanden ist.

9. Direktverpressungsverfahren nach Anspruch 7, bei dem das Füllmittel ausgewählt ist aus der Gruppe bestehend aus StarLac®, FlowLac® und Prosolv®.

10. Direktverpressungsverfahren nach einem der vorhergehenden Ansprüche, bei dem die Zusammensetzung die nachstehend aufgeführte Endformulierung hat:
| **Inhaltsstoff** | **Menge bezogen auf die Gesamtzusammensetzung** |
|---|---|
| Cinacalcet HCl | 10 - 40% |
| Quellstärke | 10 - 40% |
| hochdisperses Siliciumdioxid | 0,2 - 2 % |
| Crospovidon | 2 - 8% |
| Copovidon | 5 - 10% |
| Prosolv SMCC | 5 - 25 % |
| Starlac | 20 - 60% |
| Magnesiumstearat | 0,1 - 2,0 % |

## Revendications

1. Procédé de compression directe pour la production d'une composition pharmaceutique de cinacalcet ou de sels pharmaceutiquement acceptables de celui-ci, comprenant les étapes de :
préparation d'une composition de noyau comprenant le mélange de particules de cinacalcet ayant une valeur de distribution de tailles de particules -d(90) inférieure à 150 µm conjointement avec au moins un diluant,
tamisage de la composition de noyau pour obtenir un profil global de tailles de particules inférieur à 355 µm,
sélection séparément de matériaux de charge pour obtenir une densité apparente dans la plage allant de 350 à 650 g/l, lesdits matériaux de charge sont présents en une quantité dans la plage allant de 20 à 60 % en poids de la composition globale, et mélange de ceux-ci avec la composition de noyau dans des conditions sensiblement sèches de manière à ce que la densité apparente de la composition globale soit supérieure à 400 g/l.

2. Procédé de compression directe selon la revendication 1 dans lequel le procédé comprend en outre la compression directe des poudres résultantes sous la forme de comprimés oraux.

3. Procédé de compression directe selon la revendication 1 ou 2 dans lequel les particules de cinacalcet ont une distribution de tailles de particules -d(90) supérieure à 30 µm.

4. Procédé de compression directe selon la revendication 1 ou 2 dans lequel les particules de cinacalcet ont une distribution de tailles de particules -d(90) inférieure à 30 µm, et la composition comprend en outre un lubrifiant représentant jusqu'à 2 % (en p/p) de la composition globale.

5. Procédé de compression directe selon la revendication 1 ou 2 dans lequel les tailles de particules globales dans la composition de noyau sont inférieures à 250 µm.

6. Procédé de compression directe selon la revendication 1 ou 2 dans lequel le diluant est de l'amidon prégélatinisé.

7. Procédé de compression directe selon la revendication 1 ou 2 dans lequel les autres excipients sont choisis dans le groupe constitué par les charges, les liants, les délitants, les lubrifiants, les agents de glissement et les excipients traités simultanément.

8. Procédé de compression directe selon la revendication 7 dans lequel le liant est disponible en une quantité dans la plage allant de 5 % à 10 %.

9. Procédé de compression directe selon la revendication 7 dans lequel la charge est choisie dans le groupe constitué par le StarLac®, le FlowLac® et le Prosolv®.

10. Procédé de compression directe selon l'une quelconque des revendications précédentes dans lequel la composition comprend la formulation finale telle que décrite ci-dessous :
| Ingrédient | Quantité basée sur la composition totale % (en p/p) |
|---|---|
| Cinacalcet HCl | 10 - 40 % |
| Amidon prégélatinisé | 10 - 40 % |
| Silice colloïdale anhydre | 0,2 - 2 % |
| Crospovidone | 2 - 8 % |
| Copovidone | 5 - 10 % |
| Prosolv SMCC | 5 - 25 % |
| Starlac | 20 - 60 % |
| Stéarate de magnésium | 0,1 - 2,0 % |
